(19) Europäisches Patentamt
European Patent Office
Office européen des brevets

(11) EP 3 922 633 B1

(12) **FASCICULE DE BREVET EUROPEEN**

(45) Date de publication et mention de la délivrance du brevet:
**17.12.2025 Bulletin 2025/51**

(21) Numéro de dépôt: **21020307.1**

(22) Date de dépôt: **11.06.2021**

(51) Classification Internationale des Brevets (IPC):
***C07D 487/04*** (2006.01) ***C07D 491/16*** (2006.01)
***C07D 491/22*** (2006.01) ***C11B 9/00*** (2006.01)

(52) Classification Coopérative des Brevets (CPC):
**C07D 491/16; C07D 487/04; C07D 491/22**

(54) **PROCÉDÉ D'EXTRACTION DES ALCALOÏDES PYRROLIZIDINIQUES**

VERFAHREN ZUR EXTRAKTION VON PYRROLIZIDINALKALOIDEN

METHOD FOR EXTRACTING PYRROLIZIDINE ALKALOIDS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **11.06.2020 FR 2006128**

(43) Date de publication de la demande:
**15.12.2021 Bulletin 2021/50**

(73) Titulaire: **Robertet S.A.**
**06130 Grasse (FR)**

(72) Inventeurs:
- **Brevard, Hughes**
  **06130 Grasse (FR)**
- **Filippi, Jean-Jacques**
  **06130 Grasse (FR)**
- **Nemetz, Jörn**
  **24229 Dänischenhagen (DE)**
- **Giera, David Sebastian**
  **50769 Köln (DE)**

(74) Mandataire: **Santarelli**
**Tour Trinity**
**1 bis Place de la Défense**
**92400 Courbevoie (FR)**

(56) Documents cités:
**EP-A1- 0 508 330** **WO-A1-2020/015983**
**US-A1- 2018 303 890**

- **KOPP THOMAS ET AL: "Extracting and Analyzing Pyrrolizidine Alkaloids in Medicinal Plants: A Review", TOXINS, vol. 12, no. 5, 13 May 2020 (2020-05-13), CH, pages 320, XP055838954, ISSN: 2072-6651, DOI: 10.3390/toxins12050320**
- **MIHAJILOV-KRSTEV TATJANA ET AL: "Phytochemistry, Toxicology and Therapeutic Value of Petasites hybridus Subsp. Ochroleucus (Common Butterbur) from the Balkans", PLANTS, vol. 9, no. 6, 31 May 2020 (2020-05-31), pages 700, XP055838984, ISSN: 2223-7747, DOI: 10.3390/plants9060700**
- **ANTONELLA MAGGIO ET AL: "Chemical composition of the essential oil of Jacobaea maritima (L.) Pelser & Meijden and Jacobaea maritima subsp. bicolor (Willd.) B. Nord. & Greuter (Asteraceae) collected wild in Croatia and Sicily, respectively", NATURAL PRODUCT RESEARCH, vol. 29, no. 9, 23 December 2014 (2014-12-23), GB, pages 857 - 863, XP055740835, ISSN: 1478-6419, DOI: 10.1080/ 14786419.2014.991928**
- **SULEIMEN E M ET AL: "Constituent Composition and Cytotoxic Activity of Essential Oil from Senecio argunensis", CHEMISTRY OF NATURAL COMPOUNDS, CONSULTANTS BUREAU, NEW YORK, NY, US, vol. 52, no. 6, 25 October 2016 (2016-10-25), pages 1125 - 1126, XP036088366, ISSN: 0009-3130, [retrieved on 20161025], DOI: 10.1007/S10600-016-1883-1**
- **DE POOTER H. L ET AL: "The volatile fraction of Senecio glaucus subsp. Coronopifolius", FLAVOUR AND FRAGRANCE JOURNAL., vol. 1, no. 4-5, 1 September 1986 (1986-09-01), GB, pages 159 - 163, XP055839074, ISSN: 0882-5734, DOI: 10.1002/ffj.2730010406**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).





**(Cont. page suivante)**

- MIRZA M. ET AL: "Chemical Composition of the Essential Oil of Senecio leucostachys Baker", vol. 11, no. 2, 1 January 2008 (2008-01-01), IN, pages 179 - 183, XP055839075, ISSN: 0972-060X, Retrieved from the Internet <URL:https://www.tandfonline.com/doi/pdf/10.1080/0972060X.2008.10643616?needAccess=true> DOI: 10.1080/0972060X.2008.10643616
- MAJID MOHAMMADHOSSEINI ET AL: "Chemical Composition of the Essential Oil from Aerial Parts of Senicio gallicus Chaix Growing Wild in Iran", JOURNAL OF ESSENTIAL OIL-BEARING PLANTS, vol. 13, no. 6, 1 January 2010 (2010-01-01), IN, pages 704 - 709, XP055740834, ISSN: 0972-060X, DOI: 10.1080/0972060X.2010.10643882
- CHALCHAT JEAN-CLAUDE ET AL: "Essential Oil of Senecio squalidus L., Asteraceae", vol. 16, no. 3, 1 May 2004 (2004-05-01), UK, pages 227 - 228, XP055839079, ISSN: 1041-2905, Retrieved from the Internet <URL:https://www.tandfonline.com/doi/pdf/10.1080/10412905.2004.9698704?needAccess=true> DOI: 10.1080/10412905.2004.9698704
- MAUZ CH ET AL: "Methode zur Entfernung von Pyrrolizidin-Alkaloiden aus Arzneipflanzenextrakten", PHARMACEUTICA ACTA HELVETIAE, ELSEVIER BV, NETHERLANDS, vol. 60, 1 January 1985 (1985-01-01), pages 256 - 259, XP009133101, ISSN: 0031-6865
- AYDIN AHMET ALPER ET AL: "The medical plant butterbur (Petasites): Analytical and physiological (re)view", JOURNAL OF PHARMACEUTICAL AND BIOMEDICAL ANALYSIS, vol. 75, 1 March 2013 (2013-03-01), AMSTERDAM, NL, pages 220 - 229, XP055839002, ISSN: 0731-7085, DOI: 10.1016/j.jpba.2012.11.028

## Description

L'invention concerne un procédé d'extraction des alcaloïdes pyrrolizidiniques présents dans un extrait végétal d'intérêt.

Les alcaloïdes pyrrolizidiniques (APs) forment une classe d'alcaloïdes et de métabolites secondaires, caractérisés par une structure pyrrolizidine formée de deux cycles pyrroles. Ces alcaloïdes sont produits par les plantes et constituent un mécanisme de défense contre les animaux herbivores.

Il n'a été trouvé aucune application thérapeutique à ces alcaloïdes et c'est plutôt leur toxicité qui retient l'attention.

Il est soupçonné que les extraits de plantes utilisables en parfumerie, cosmétique, pharmacie, voire en alimentation, pourraient contenir des alcaloïdes pyrrolizidiniques. Ces impuretés naturelles sont des contaminants particulièrement indésirables en raison de leur toxicité élevée. De ce fait, les préparations à base de plantes, en général, sont sous surveillance et une dose quotidienne ingérée de 1,5µg de ces impuretés semble être une valeur qui ne présente pas de risques carcinogéniques.

Les autorités de certains pays, particulièrement européens, ont publié une déclaration sur l'analyse des alcaloïdes pyrrolizidiniques afin de garantir la qualité et la sécurité des médicaments, déclaration qui mentionnait une liste de plantes dans lesquelles des alcaloïdes pyrrolizidiniques ont été mesurés. Les concentrations sont généralement faibles, de l'ordre de quelques µg/kg:

Le millepertuis (*Hyperici herba*),
La fleur de la passion (*Passiflorae herba*),
La camomille (*Matricariae flos*),
Le manteau de dame (*Alchemillae herba*),
La réglisse (*Liquiritiae radix*),
La mélisse (*Melissae folium*),
La menthe poivrée (*Menthae piperitae folium*),
La sauge (*Salviae folium*),
Le pissenlit et racine (*Taraxaci herba cum radice*).

Il est cependant notable que les alcaloïdes pyrrolizidiniques sont susceptibles de se retrouver dans tous les végétaux et que donc l'invention ne s'adresse pas aux seuls végétaux et/ou extraits desdits végétaux listés précédemment mais à tous les végétaux et/ou extraits susceptibles de comprendre des alcaloïdes pyrrolizidiniques.

La structure générale des alcaloïdes pyrrolizidiniques comprend un motif pyrrolizidine ou 1*H*-Pyrrolizine, hexahydro- (système cyclique 5/5 condensé avec un atome d'azote sur une tête de pont, Formule 1) appelé nécine (base) et substitué par un groupe hydroxyméthyle en C-1 et un groupe hydroxyle en C-7 (Formule I).

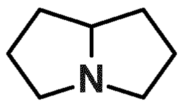

Formule I

Lorsque la pyrrolizidine est substituée par un groupe hydroxyméthyle en C-1 et un groupe hydroxyle en C-7 (Formule II), celle-ci prend le nom de nécine, ou encore rétronécine lorsque celle-ci est insaturée en position-1,2. C'est l'unité de base commune à tous les alcaloïdes pyrrolizidiniques.

Les deux fonctions hydroxyles sont généralement estérifiées avec un ou deux acide(s) appelés acides néciques. Ces deux chaînes latérales peuvent également se cycliser en formant une structure macrocyclique.

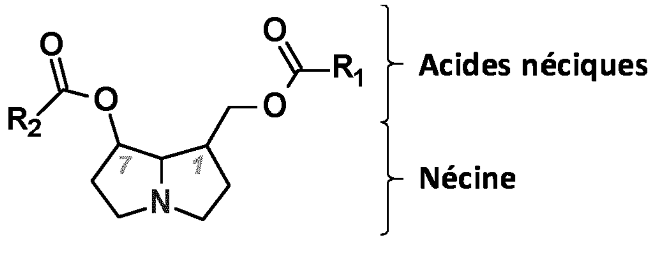


Formule II

À ce jour, plus de 370 structures individuelles d'alcaloïdes pyrrolidiziniques provenant de plus de 560 espèces de plantes ont été isolées. Compte tenu des études chimio-systématiques et biogénétiques, la majorité des alcaloïdes pyrrolizidiniques connus appartiennent à l'un des quatre types de squelette de base dont les structures moléculaires III, IV, V et VI sont illustrées ci-après.

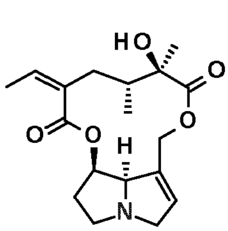

Senecionine-type
(> 100 structures)

Formule III

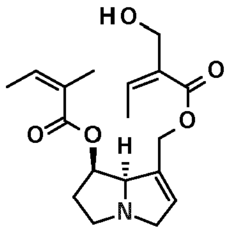

Triangularine-type
(> 50 structures)

Formule IV

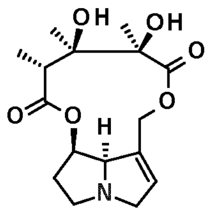

Monocrotaline-type
(> 30 structures)

Formule V

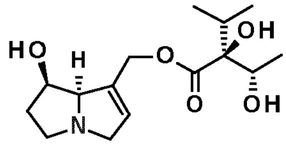

Lycopsamine-type
(> 100 structures)

Formule VI

En outre, la rupture de la liaison intracyclique N-C commune à tous les alcaloïdes pyrrolizidiniques peut être obtenue par synthèse pour conduire à un cinquième squelette d'alcaloïde pyrrolizidinique de type otonécine (Formule VII) comme par exemple, la senkirkine.

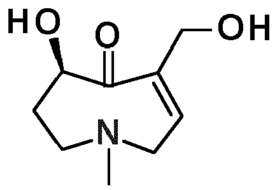

Formule VII

Le cinquième type englobe les phalaénopsines (alcaloïdes pyrrolizidiniques d'orchidées) rares et structurellement plus simples, auxquelles il manque la double liaison 1,2 et le groupement hydroxyle en position 7 sur le squelette de base de la nécine (Formule VIII).

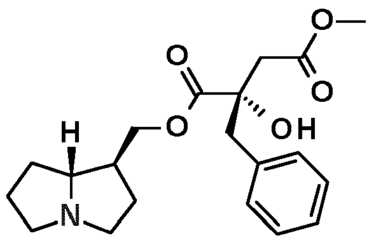

Formule VIII

Une étude européenne réalisée sur plus d'un millier d'échantillon a permis de mettre en évidence 35 alcaloïdes pyrrolizidiniques dans les échantillons d'origine animale et 28 alcaloïdes pyrrolizidiniques dans les échantillons d'origine végétale listés dans le tableau 1 suivant. Ce sont ces 28 alcaloïdes pyrrolizidiniques qui sont pris en compte dans le contrôle de la qualité des extraits de plantes destinés à être utilisés en pharmacie, en alimentation, en cosmétique ou en parfumerie.

Tableau 1 : liste des 28 alcaloïdes pyrrolizidiniques suivis dans les échantillons d'origine végétale

| | | | |
|---|---|---|---|
| 1. | Echimidine | 15. | Lycopsamine |
| 2. | Echimidine-N-oxide | 16. | Lycopsamine-N-oxide |
| 3. | Erucifoline | 17. | Monocrotaline |
| 4. | Erucifoline-N-oxide | 18. | Monocrotaline-N-oxide |

(suite)

| | | | |
|---|---|---|---|
| 5. | Europine | 19. | Retrorsine |
| 6. | Europine-N-oxide | 20. | Retrorsine-N-oxide |
| 7. | Heliotrine | 21. | Senecionine |
| 8. | Heliotrine-N-oxide | 22. | Senecionine-N-oxide |
| 9. | Intermedine | 23. | Seneciphylline |
| 10. | Intermedine-N-oxide | 24. | Seneciphylline-N-oxide |
| 11. | Jacobine | 25. | Senecivernine |
| 12. | Jacobine-N-oxide | 26. | Senecivernine-N-oxide |
| 13. | Lasiocarpine | 27. | Senkirkine |
| 14. | Lasiocarpine-N-oxide | 28. | Trichodesmine |

Tous sont des représentants des quatre types de structure de base répondant aux formules III, IV, V et VI.

Malgré la diversité structurelle apparente des alcaloïdes pyrrolizidiniques, leurs propriétés chimiques sont très similaires.

Outre le fait que les 28 alcaloïdes pyrrolizidiniques mentionnés plus haut appartiennent à quatre types de base différents, ils portent tous des chaînes latérales hydrocarbonées avec des fonctions polaires, principalement des groupes hydroxyle, méthoxyle et ester, et leurs poids moléculaires sont supérieurs ou égaux à 300 Da. Ainsi, en termes de polarité et de volatilité, qui sont les caractéristiques clés déterminant dans quelle mesure une substance est entrainable à la vapeur, les propriétés chimiques de tous les alcaloïdes pyrrolizidiniques sont très étroitement liées et contrastent de manière frappante avec les propriétés des composants généralement présents dans les extraits de plantes, particulièrement les huiles essentielles, absolues, concrètes ou résinoïdes.

Les alcaloïdes pyrrolizidiniques sont principalement rencontrés dans deux familles botaniques, les Boraginaceae et les Asteraceae, et accessoirement chez les Fabaceae, les Apocynaceae, les Euphorbiaceae, les Orchidaceae, et les Poaceae.

En ce qui concerne les propriétés chimiques, les alcalcaloïdes de pyrrolizidine constituent un groupe relativement homogène.

La plupart des alcaloïdes pyrrolizidiniques sont mutagènes et inducteurs de tumeurs hépatiques.

Il a été montré chez le rat que des alcaloïdes pyrrolizidiniques comme les rétrorsine, senkirkine, monocrotaline, lasiocarpine et symphytine et plusieurs plantes (*Tussilago farfara L., Symphytum officinale L., Petasites jalcaloïdes pyrrolizidiniques onicus Maxim.* etc.) pouvaient provoquer des tumeurs hépatiques lorsqu'ils sont administrés régulièrement par voie orale. Il a aussi été prouvé expérimentalement que plusieurs alcaloïdes du groupe étaient mutagènes et tératogènes.

Les alcaloïdes pyrrolizidiniques macrocycliques de type III et V (sénécionine, rétrorsine, sénéciphylline, ridelline) sont les plus toxiques. Puis viennent les diesters, qui sont plus toxiques que les monoesters.

Une consommation régulière de végétaux contenant des alcaloïdes pyrrolizidiniques peut être responsable de graves intoxications hépatiques. Une intoxication par ces alcaloïdes pyrrolizidiniques peut se traduire par une perte d'appétit, des douleurs, une distension abdominale, une augmentation du volume du foie (hépatomégalie).

A titre d'exemple, on peut citer la consoude (*Symphytum officinale*) qui contient des alcaloïdes pyrrolizidiniques comme l'intermédine, la lycopsamine, la 7-acétyl-intermédine et qui en raison de leur toxicité, voit son usage interdit dans de nombreux pays.

En Guadeloupe, une plante commune, la sonnette (*Crotalaria retusa* L.) sert à confectionner un "thé de sonnette" remède populaire contre beaucoup d'indispositions. Cependant des cas d'intoxication sévère ont été décrits. Plusieurs alcaloïdes pyrrolizidiniques ont été identifiés dans *Crotalaria retusa* L. principalement la monocrotaline, mais également rétronécine-N-oxyde, rétusine, rétusamine. La monocrotaline, présente surtout dans les graines, est réputée être hépatotoxique, neurotoxique et génotoxique. Cette molécule et ses métabolites ont la capacité de franchir la barrière hémato-encéphalique. *Crotalaria retusa* L. est notamment responsable (avec *C. crispata*) de la maladie de Kimberley qui affecte les chevaux dans le nord de l'Australie (précisément dans la région du Kimberley).

Les autres "plantes médicinales" contenant ces composés hépatotoxiques sont le tussilage, la bourrache, les héliotropes, les cynoglosses, les séneçons etc.

Des alcaloïdes pyrrolizidiniques ont été identifiés dans les herbes médicinales de Chine, d'Amérique du Sud et du Sri Lanka.

De grands épisodes d'empoisonnement ont été décrits en Afghanistan, en Inde et dans l'ancienne URSS du fait

de la contamination des récoltes de blé par des Boraginacées (*Heliotropium lasiocarpum, H. popovii, H. europaeum*).

On comprend de ce qui précède qu'il est recherché de disposer d'un moyen rapide, efficace et sûr d'éliminer les alcaloïdes pyrrolizidiniques potentiellement présents dans des extraits de plantes utilisables en pharmacie, en alimentation, en cosmétique ou en parfumerie.

C'est un des buts de la présente invention.

En effet, la Demanderesse a montré qu'une étape d'hydrodistillation d'une composition organique contenant des alcaloïdes pyrrolizidiniques permet d'éliminer ces derniers de la composition.

Tel que montré par la Demanderesse, il s'avère qu'au cours de l'hydrodistillation de phases organiques comprenant des alcaloïdes pyrrolizidiniques, comme par exemple pourraient l'être des huiles essentielles exprimées ou hydrodistillées, les alcaloïdes pyrrolizidiniques ne sont pas entrainés par la vapeur d'eau et restent piégés dans la phase aqueuse.

De ce fait, l'hydrodistillation constitue une méthode de choix pour éliminer les alcaloïdes pyrrolizidiniques éventuellement présents dans une composition organique.

En particulier, la présence potentielle des alcaloïdes pyrrolizidiniques dans les huiles essentielles, particulièrement dans les huiles essentielles exprimées, serait ainsi éliminée par une étape d'hydrodistillation supplémentaire.

Ainsi, selon un premier aspect l'invention vise à traiter un extrait végétal, ledit extrait végétal d'intérêt étant une huile essentielle, susceptible de contenir des alcaloïdes pyrrolizidiniques, par hydrodistillation en vue de l'élimination desdits alcaloïdes pyrrolizidiniques présents dans les produits de départ par dissolution/concentration dans une phase aqueuse (résidus de distillation ou hydrolat).

L'invention a donc pour objet un procédé d'extraction des alcaloïdes pyrrolizidiniques présents dans un extrait végétal d'intérêt, ledit extrait végétal d'intérêt étant une huile essentielle, comprenant au moins une étape d'hydrodistillation.

Par « extraction des alcaloïdes pyrrolizidiniques présents dans un extrait végétal d'intérêt », on entend la séparation, totale ou partielle, des alcaloïdes pyrrolidiziniques présents dans ledit extrait végétal d'intérêt en vue de les retirer dudit extrait végétal.

Selon l'invention, l'extrait de départ, duquel il est souhaité de retirer les alcaloïdes pyrrolizidiniques, est une huile essentielle.

Selon une variante de l'invention, l'extrait à traiter peut être traité à la vapeur d'eau pour entrainer les substances volatiles contenues dans ledit extrait à traiter. La vapeur ainsi formée peut être ensuite dirigée vers un condensateur et le liquide ainsi récolté peut se séparer en deux phases :

- une phase supérieure, plus légère que l'eau qui contient les substances volatiles, et
- une phase inférieure, la phase aqueuse, qui peut être redirigée (ou non) vers le bouilleur pour être remise en circulation afin d'éliminer les éventuelles substances volatiles résiduelles par un ou plusieurs même(s) cycle(s) d'évaporation et/ou de condensation et/ou de séparation.

Selon la variante du procédé, il est possible d'ajouter un solvant organique apolaire dans les condensats obtenus au cours du traitement à la vapeur d'eau (hydrodistillation), ce qui peut permettre d'obtenir une meilleure décantation.

Selon cette variante, le solvant peut être choisi parmi les hydrocarbures linéaires, ramifiés ou cycliques préférentiellement parmi le pentane, l'hexane, l'heptane, le trimethylpentane, le cyclohexane. Ces solvants peuvent être utilisés seuls ou encore en mélange avec l'éther diéthylique. Dans le cas d'un mélange avec l'éther diéthylique, celui-ci peut être ajouté dans une quantité comprise entre 25% et 75% du volume dudit solvant organique apolaire, de préférence 50%, exprimé en pourcentage volumique.

Encore selon cette variante, le solvant peut être ajouté dans une quantité comprise entre 5% et 20% du volume initial à traiter, préférentiellement entre 10% et 15%, exprimé en pourcentage volumique.

L'extrait à traiter, débarrassé des alcaloïdes pyrrolizidiniques, peut être alors récupéré et la phase aqueuse contenant les alcaloïdes pyrrolizidiniques peut être éliminée.

D'autres avantages et particularités de l'invention apparaitront dans les exemples non limitatifs présentés ci-après, ainsi que dans les figures annexées dans lesquelles :

La Figure 1 présente les profils UPLC-MS d'une huile essentielle d'Orange dopée en APs (a), la même essence d'Orange dopée en APs, distillée (b), l'huile essentielle d'Orange brute (c) et l'huile essentielle d'Orange brute distillée (d).

La Figure 2 présente les profils UPLC-MS d'une huile essentielle d'Eucalyptus dopée en APs (a), la même essence d'Eucalyptus dopée en APs, distillée (b), l'huile essentielle d'Eucalyptus brute (c) et l'huile essentielle d'Eucalyptus brute distillée (d).

## Exemples

**Exemple 1** : **Traitement d'échantillon d'huiles essentielles *d'Eucalyptus globulus* Labill., de *Citrus sinensis* (L.) Osbeck et *de Citrus x limon* (L.) Burm. f.**

**Mode opératoire**

Dans un ballon de 500 mL, on introduit 300 mL d'eau distillée, un barreau magnétique, quelques pierres ponces et une quantité pesée d'échantillon entre 15 et 30 g.
Le ballon est alors raccordé à un appareil de type Clevenger et placé dans un bain d'huile chauffant.
On ajoute 10 mL d'eau distillée et 4 à 5 mL de pentane afin de remplir le séparateur.
On ouvre l'arrivée d'eau pour le réfrigérant et on porte à reflux pendant deux heures.
On arrête alors le chauffage et on laisse décanter. La phase organique et la phase aqueuse décantent dans le séparateur et sont recueillies.
**ANALYSES. Conditions HR-TOF-MS** : Les analyses ont été effectuées sur un système Waters XEVO G2 TOF. Conditions de source ESI : tension capillaire : 0,5 kV, tension de cône : 45 V, cône d'extraction : 4 V, température de source : 120 °C, température de désolvatation : 400 °C, débit de gaz : 10 L/h, débit de gaz de désolvatation : 1200 L/h. Les analyses ont été effectuées en mode positif. Le spectromètre de masse a été étalonné avec du formiate de sodium pour une gamme de masse allant de 50 à 1200 Da. La Leucine-Enképhaline (lockmass) est le standard utilisé pour corriger les masses. Les deux masses caractéristiques de la Leucine-Enképhaline ont été vérifiées (278,1141; 556,2771). Les acquisitions ont été réalisées en mode MSE en utilisant l'argon comme gaz de collision. **Fragmentation** : Le mode MSE permet l'acquisition simultanée de spectres de masse à l'énergie de collision faible et élevée, le premier permettant d'obtenir un ion parent, le dernier de produire des ions fragments.

**EXPERIMENTAL**

Après dilution des extraits d'huile essentielle dans du méthanol, les alcaloïdes pyrrolizidiniques sont chromatographiés par système RP-UPLC, en mode gradient d'élution, détectés et identifiés par spectrométrie de masse et quantifiés par une méthode d'étalonnage externe.

**Substances de référence**

Les différents alcaloïdes pyrrolizidiniques et leurs oxydes sont obtenus auprès de Sigma-Aldrich. Leur pureté est donnée à 95 % minimum.

**Solutions de référence**

Des solutions standards d'alcaloïdes pyrrolizidiniques sont préparées comme suit : Dans une fiole jaugée de 100 mL (Vf), on pèse exactement 10 mg (Ms) des substances étalons et on ajuste avec du méthanol. Les solutions sont homogénéisées avec un bain à ultrasons pendant 3 min.
La concentration de la solution standard est donnée par l'équation suivante :

$$C\ (mg/mL) = (Ms \times Pu)/(100 \times Vf)$$

Ms = Poids de la substance standard
Pu = Pureté de la substance étalon en pourcentage

Ensuite, cette solution mère est diluée jusqu'à obtenir les concentrations appropriées pour la sensibilité du détecteur.
La solution standard sera préparée pour chaque étalonnage de la méthode quantitative.

**Préparation des échantillons**

Dans une fiole jaugée de 20 mL, on pèse exactement 1 g (Pe) de l'échantillon et on ajuste avec du méthanol. La solution est homogénéisée pendant 3 min avec un bain à ultrasons, puis filtrée à l'aide d'une seringue sur un filtre en PTFE de 0,25 μm, puis transférée dans un flacon d'injection.
Si nécessaire, cette solution doit être successivement diluée pour tomber dans une plage de linéarité appropriée du détecteur.

**Exemple 2 : Etude de la présence ou l'absence d'alcaloïdes pyrrolizidiniques dans des échantillons d'huile essentielle enrichis en alcaloïdes pyrrolizidiniques avant et après hydrodistillation**

Afin de démontrer le principe de l'invention, une étude quantitative destinée à montrer la présence ou l'absence d'alcaloïdes pyrrolizidiniques dans des échantillons d'huile essentielle enrichis en alcaloïdes pyrrolizidiniques a été réalisée avant et après hydrodistillation.

Dans cette étude, on a utilisé des alcaloïdes pyrrolizidiniques disponibles commercialement et représentant deux classes précédemment décrites de type II et IV, à savoir : alcaloïde type cyclique (classe la plus représentée) pour la sénécionine [130-01-8], sénécionine-N-oxyde [13268-67-2], rétrorsine [480-54-6], rétrorsine-N-oxyde [15503-86-3], jacobine [3870-67-3], jacobine-N-oxyde [38710-25-7], sénéciphylline [480-81-9], sénéciphylline-N-oxyde [38710-26-8], et alcaloïde type mono-ester pour la lycopsamine [10285-07-1], lycopsamine-N-oxyde [95462-15-0], intermédine [10285-06-0], intermédine-N-oxyde [95462-14-9].

**Préparation des échantillons enrichis en alcaloïdes pyrrolizidiniques**

Trois huiles essentielles de citron, d'orange et d'eucalyptus, dopées jusqu'à 100 ppm de chacun des alcaloïdes pyrrolizidiniques et de leurs oxydes respectifs, ont été hydrodistillées séparément pendant deux heures selon le protocole décrit à l'exemple 1. L'expérimentation est réalisée en duplicat.

Avant et après hydrodistillation, les phases organique et aqueuse sont séparées et injectées en UPLC-HRMS/TOF.

Les résultats sont rapportés dans le tableau 2 ci-dessous.

Tableau 2 : Fragmentation des APs et de leurs oxydes respectifs obtenue en UPLC-HRMS

| **Composé RN CAS** | **Formule moléculaire** | **Masse exacte** | **[M+H]$^+$** | **[M-H]-** | **[M+NH4]$^+$** | **[M+Na]$^+$** | **Ion fils 1** | **Ion fils 2** |
|---|---|---|---|---|---|---|---|---|
| **Intermedine** 10285-06-0 | C15H25NO5 | 299.173279 | 300.18110 | 298.16545 | 317.20765 | 322.16305 | 156.3 | 138.3 |
| **Intermedine-N-oxide** 95462-14-9 | C15H25NO6 | 315.168182 | 316.17601 | 314.16036 | 333.20255 | 338.15795 | | |
| **Lycopsamine** 10285-07-1 | C15H25NO5 | 299.173279 | 300.18110 | 298.16545 | 317.20765 | 322.16305 | 156.3 | 138.3 |
| **Lycopsamine-N-oxide** 95462-15 | C15H25NO6 | 315.168182 | 316.17601 | 314.16036 | 333.20255 | 338.15795 | | |
| **Monocrotaline** 315-22-0 | C16H23NO6 | 325.152527 | 326.16035 | 324.14470 | 343.18690 | 348.14230 | 237.3 | 120.3 |
| **Monocrotaline-N-oxide** 35337-98-5 | C16H23NO7 | 341.14745 | 342.15528 | 340.13963 | 359.18182 | 364.13722 | 137.4 | 118.3 |
| **Retrosine** 480-54-6 | C18H25NO6 | 351.168182 | 352.17601 | 350.16036 | 369.20255 | 374.15795 | 138.3 | 120.3 |
| **Retrosine-N-oxide** 15503-86-3 | C18H25NO7 | 367.1631 | 368.17093 | 366.15528 | 385.19747 | 390.15287 | 136.2 | 118.2 |
| **Senecionine** 130-01-8 | C18H25NO5 | 335.173279 | 336.18110 | 334.16545 | 353.20765 | 358.16305 | 138.2 | 120.2 |
| **Senecionine-N-oxide** 13268-67-2 | C18H25NO6 | 351.16819 | 352.17602 | 350.16037 | 369.20256 | 374.15796 | 136.3 | 118.1 |
| **Seneciphylline** 480-81-9 | C18H23NO5 | 333.157623 | 334.16545 | 332.14980 | 351.19199 | 356.14739 | 138.4 | 120.3 |

(suite)

| Composé RN CAS | Formule moléculaire | Masse exacte | $[M+H]^+$ | [M-H]- | $[M+NH4]^+$ | $[M+Na]^+$ | Ion fils 1 | Ion fils 2 |
|---|---|---|---|---|---|---|---|---|
| **Seneciphylline-N-oxide** 38710-26-8 | C18H23NO6 | 349.15254 | 350.16037 | 348.14472 | 367.18691 | 372.14231 | | |

Les résultats montrent l'absence des signaux correspondant à la sénécionine, sénécionine-N-oxyde, rétrorsine, rétrorsine-N-oxyde, jacobine, jacobine-N-oxyde, séneciphylline, séneciphylline-N-oxyde, lycopsamine, lycopsamine-N-oxyde, intermédine, intermédine-N-oxyde dans la phase organique recueillie après hydrodistillation. (Tableau 2).

Ces expériences confirment les hypothèses initiales. Les alcaloïdes pyrrolizidiniques sont des substances classées dans la catégorie des petites molécules selon CAS. Néanmoins, avec des masses moléculaires supérieures à 300 DA, ainsi que des points d'ébullition élevés, une forte polarité, une forte solubilité et une très faible pression de vapeur, ces paramètres constituent un frein à leur transfert dans la phase huileuse pendant le procédé d'entrainement à la vapeur.

La quasi-totalité des alcaloïdes pyrrolizidiniques ajoutés dans la phase organique est retrouvée dans la phase aqueuse comme le montrent les résultats rapportés dans les tableaux 3 et 4.

Tableau 3 : Huile essentielle de Citron dopée - Dosage des APs sur le distillat et la phase aqueuse correspondante

| | **φ Organique (distillat)** | **φ Aqueuse (résidu de distillation)** | | **φ Organique (distillat)** | **φ Aqueuse (résidu de distillation)** | |
|---|---|---|---|---|---|---|
| | C [ppm] | CT [ppm] | C [ppm] | C [ppm] | CT [ppm] | C [ppm] |
| **1** | **< LoQ** | 0,806 | **0,810** | **< LoQ** | 0,868 | **0,839** |
| **2** | **< LoQ** | 0,662 | **0,691** | **< LoQ** | 0,621 | **0,605** |
| **3** | **< LoQ** | 0,656 | **0,678** | **< LoQ** | 0,626 | **0,698** |

*1 : Rétrorsine ; 2 : Rétrorsine-N-oxyde; 3 : Lycopsamine*
*C : Concentration ; CT : Concentration théorique*
*LOQ : 2 ppb*

Tableau 4 : Huile essentielle de Citron dopée - Dosage des APs sur le distillat et la phase aqueuse correspondante

| | **φ Organique (distillat)** | **φ Aqueuse (résidu de distillation)** | | **φ Organique (distillat)** | **φ Aqueuse (résidu de distillation)** | |
|---|---|---|---|---|---|---|
| | C [ppm] | CT [ppm] | C [ppm] | C [ppm] | CT [ppm] | C [ppm] |
| **1** | **< LoQ** | 0.797 | **0.663** | **< LoQ** | 0.828 | **0.711** |
| **2** | **< LoQ** | 0.457 | **0.678** | **< LoQ** | 0.593 | **0.612** |
| **3** | **< LoQ** | 0.550 | **0.588** | **< LoQ** | 0.597 | **0.621** |
| **4** | **< LoQ** | 0.028 | 0.024 | **< LoQ** | 0.030 | 0.027 |

*1 : Rétrorsine ; 2 : Rétrorsine-N-oxyde; 3 : Lycopsamine ; 4 : Lycopsamine-N-oxyde*
*C : Concentration ; CT : Concentration théorique*
*LOQ : 2 ppb*

Les profils UPLC-MS ciblés sur la rétrorsine et son oxyde, et sur la lycopsamine (figure 1, figure 2) montrent que ces substances sont absentes dans l'huile essentielle de citron brute et distillée, mais aussi dans l'huile essentielle avec standards et distillée. Il en est de même pour les essences d'orange et d'eucalyptus.

## Revendications

1. Procédé d'extraction des alcaloïdes pyrrolizidiniques présents dans un extrait végétal d'intérêt en vue de les retirer dudit extrait végétal, ledit extrait végétal d'intérêt étant une huile essentielle, comprenant au moins une étape d'hydrodistillation.

2. Procédé selon la revendication 1, comprenant les étapes suivantes :

- traitement de l'huile essentielle à la vapeur d'eau pour entrainer les substances volatiles,
- direction de la vapeur ainsi formée vers un condensateur, le liquide ainsi récolté étant séparé en deux phases :

  • une phase supérieure, qui contient les substances volatiles, et
  • une phase inférieure, la phase aqueuse, qui est, de manière optionnelle, remise en circulation afin d'éliminer les éventuelles substances volatiles résiduelles par un ou plusieurs cycle(s) d'évaporation et/ou de condensation et/ou de séparation.

3. Procédé selon la revendication 2, dans lequel on ajoute un solvant organique apolaire dans les condensats.

4. Procédé selon la revendication 3, dans lequel le solvant organique apolaire est choisi parmi les hydrocarbures linéaires, ramifiés ou cycliques préférentiellement parmi le pentane, l'hexane, l'heptane, le trimethylpentane et le cyclohexane.

5. Procédé selon l'une des revendications 3 ou 4, dans lequel le solvant organique apolaire peut être additionné d'éther d'éthylique dans une quantité comprise entre 25% et 75%, de préférence 50%, dudit solvant organique apolaire, en pourcentage volumique.

6. Procédé selon l'une des revendications 2, 3 ou 4, dans lequel le solvant organique est ajouté dans une quantité comprise entre 5% et 20% du volume initial à traiter, de préférence entre 10% et 15%, en pourcentage volumique.

**Patentansprüche**

1. Verfahren zur Extraktion der in einem Pflanzenextrakt von Interesse vorhandenen Pyrrolizidinalkaloide, um sie aus dem Pflanzenextrakt zu entfernen, wobei der Pflanzenextrakt von Interesse ein ätherisches Öl ist, das mindestens einen Schritt der Hydrodestillation umfasst.

2. Verfahren nach Anspruch 1, umfassend die folgenden Schritte:

- Behandeln des ätherischen Öls mit Wasserdampf, um flüchtige Stoffe zu entziehen,
- Richten des so gebildeten Dampfes zu einem Kondensator, wobei die so gesammelte Flüssigkeit in zwei Phasen getrennt wird:

  • eine obere Phase, die flüchtige Stoffe enthält, und
  • eine untere Phase, die wässrige Phase, die optional wieder in Umlauf gebracht wird, um eventuelle flüchtige Reststoffe durch einen oder mehrere Verdampfungs- und/oder Kondensations- und/oder Trennungszyklen zu entfernen.

3. Verfahren nach Anspruch 2, wobei dem Kondensat ein nichtpolares organisches Lösungsmittel hinzugefügt wird.

4. Verfahren nach Anspruch 3, wobei das nichtpolare organische Lösungsmittel aus linearen, verzweigten oder zyklischen Kohlenwasserstoffen, bevorzugt aus Pentan, Hexan, Heptan, Trimethylpentan und Cyclohexan, ausgewählt ist.

5. Verfahren nach einem der Ansprüche 3 oder 4, wobei dem nichtpolaren organischen Lösungsmittel Ethylether in einer Menge zwischen 25 % und 75 %, vorzugsweise 50 %, des nichtpolaren organischen Lösungsmittels in Volumenprozent zugesetzt werden kann.

6. Verfahren nach einem der Ansprüche 2, 3 oder 4, wobei das organische Lösungsmittel in einer Menge zwischen 5 % und 20 % des zu behandelnden Ausgangsvolumens, vorzugsweise zwischen 10 % und 15 %, in Volumenprozent hinzugefügt wird.

## Claims

1. A method for extracting pyrrolizidine alkaloids present in a plant extract of interest with a view to removing them from said plant extract, said plant extract of interest being an essential oil, comprising at least one hydrodistillation step.

2. The method according to claim 1, comprising the following steps of:

   - treating the essential oil with steam to entrain the volatile substances,
   - directing the steam thus formed towards a condenser, the liquid thus collected being separated into two phases:

     • an upper phase, which contains the volatile substances, and
     • a lower phase, the aqueous phase, which is, optionally, recirculated in order to remove any residual volatile substances by one or more evaporation and/or condensation and/or separation cycle(s).

3. The method according to claim 2, wherein a non-polar organic solvent is added to the condensates.

4. The method according to claim 3, wherein the non-polar organic solvent is chosen from linear, branched, or cyclic hydrocarbons preferably from pentane, hexane, heptane, trimethylpentane, and cyclohexane.

5. The method according to one of claims 3 or 4, wherein the non-polar organic solvent may be supplemented with ethyl ether in a quantity between 25% and 75%, preferably 50%, of said non-polar organic solvent, by volume percentage.

6. The method according to one of claims 2, 3 or 4, wherein the organic solvent is added in a quantity between 5% and 20% of the initial volume to be treated, preferably between 10% and 15%, by volume percentage.

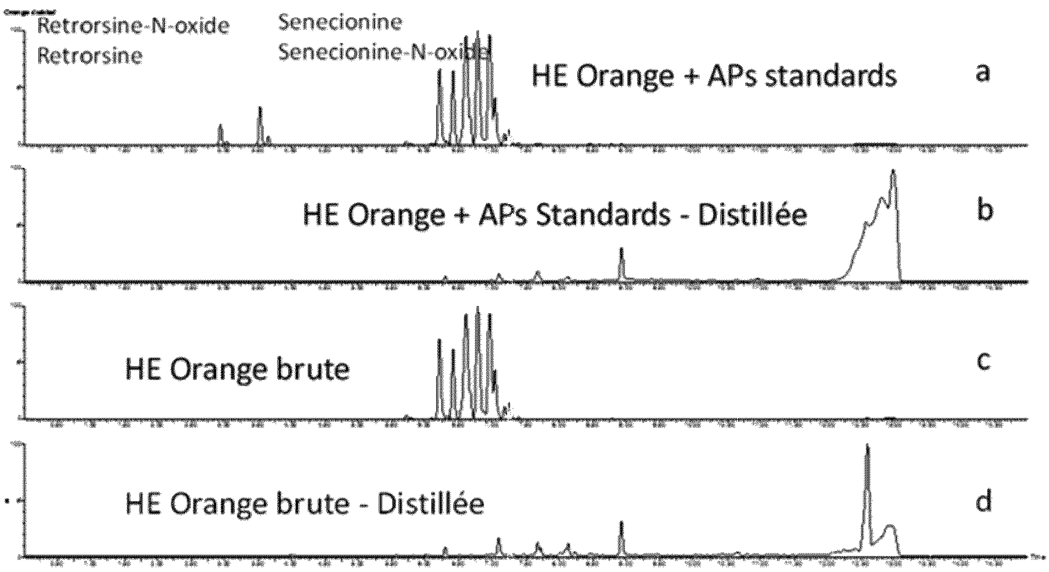


FIGURE 1

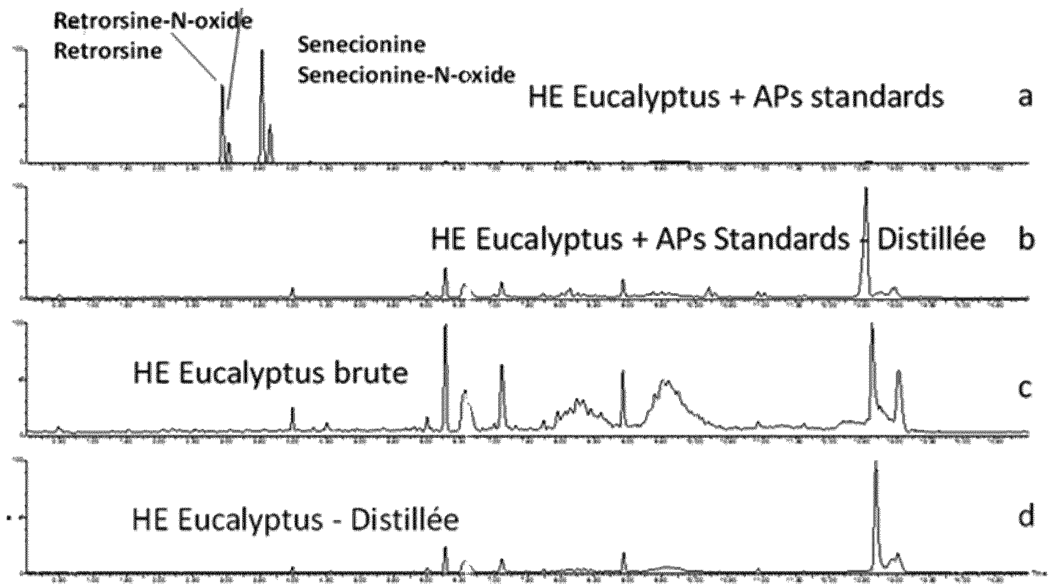


FIGURE 2

t EPO via Web-Form on Jun 11, 2021